# EUROPEAN PATENT SPECIFICATION

(11) **EP 1 146 852 B1**
(45) Date of publication and mention of the grant of the patent: **02.04.2003**
(21) Application number: 00901846.6
(22) Date of filing: 20.01.2000
(51) Int. Cl.: A61K 7/48

(54) **A COSMETIC COMPOSITION BASED ON MENTHOL AND MENTHYL LACTATE, HAVING LITTLE ODOR AND BEING NON-IRRITATING**
KOSMETISCHE ZUSAMMENSETZUNG AUF DER BASIS VON MENTHOL UND MENTHYLLACTAT, DIE EINEN GERINGEN GERUCH AUFWEIST UND NICHT REIZT
COMPOSITION COSMETIQUE A BASE DE MENTHOL ET DE LACTATE DE MENTHYLE PEU ODORANTE ET NON IRRITANTE

(30) Priority: 22.01.1999 FR 9900704
(43) Date of publication of application: 24.10.2001
(73) Proprietor: Johnson & Johnson Consumer France, 92130 Issy les Moulineaux (FR)
(72) Inventor: MAHE, Véronique, F-78740 Vaux-sur-Seine (FR); JOETZJER, Pascale, F-27000 Louviers (FR); GUISE, Anne Emmanuelle, Les Jardins de la Préfect., F-76000 Rouen (FR)
(74) Representative: Martin, Jean-Jacques
(86) International application number: IB0000149
(87) International publication number: WO00042983

(56) References cited:
- FR-A- 2 342 057
- US-A- 5 523 078
- US-A- 5 602 178
- CHEMICAL ABSTRACTS, vol. 122, no. 14, 3 April 1995 (1995-04-03) Columbus, Ohio, US; abstract no. 169701, ITO, KENZO ET AL: "Cosmetics containing menthol derivatives" XP002118337 cited in the application & JP 06 329528 A (SHISEIDO CO LTD, JAPAN) 29 November 1994 (1994-11-29)

## Description

The present invention relates to a freshening cosmetic composition characterized in that it comprises 0.01% to 2% by weight menthol and 0.1% to 10% by weight menthyl lactate, the menthol / menthyl lactate ratio lying in the range 1/1 to 1/10, and being such that the odor of the menthol is barely perceptible, said composition possessing the advantage of not being irritating, in particular for the sensitive parts of the human body, while conserving the various beneficial effects of menthol.

Menthol is a compound well known to the person skilled in the art for its analgesic, freshening, and odoriferous properties, and it has often been used in cosmetic and pharmaceutical products. In addition, this substance is used as flavoring in numerous preparations for alimentary use or indeed in toothpastes.

As recommended by the Food and Drug Administration (FDA), an effective quantity of menthol in a composition must lie in the range 1.25% to 16%. Nevertheless, at such concentrations, the flavor and the odor of the substance are very powerful, and that is poorly appreciated by consumers. In addition, this power is illustrated by the fact that menthol has been used in industrial methods for manufacturing refrigerant products (FR 74/27045).

Concerning cosmetic compositions, there are numerous compounds capable of giving rise to a cooling effect on the skin. For example, patent DE 24 05 900 describes a composition containing menthol, camphor, and alcohol for preparing a slimming composition designed to induce metabolization of skin fat by cooling. SU 15 60 215 relates to a composition for treating gingivitis which contains an association of camphor, menthol, thymol, and salicylic acid. CN 90 10 88 731 relates to a composition for repelling mosquitos and comprising, in particular, menthol and camphor.

Compositions specifically based on menthol are described in the documents of the prior art. Nevertheless, given the overwhelming power of menthol, various esters of menthol have been prepared for the purpose of reducing the odoriferous properties of this substance, but most of such esters possess odors that are very disagreeable, making them unusable in products for local application. In order to obtain satisfactory effectiveness of their analgesic or anti-irritant action, said derivatives are required in quantities that are large. Thus, with an effective quantity of menthol derivatives, said odors constitute a serious obstacle for use as a cosmetic.

One of said derivatives, menthyl lactate, is used in pharmaceutical compositions for oral administration for treating diseases such as respiratory deficiencies, the common cold, sinusitises, bronchitises, or asthma. The beneficial properties of menthyl lactate are set out, for example, in WO 97/42945.

Menthyl lactate has also been used in cosmetic compositions for local application as a freshener. According to the manufacturers, that compound does not produce the mint odor which characterizes most other menthol derivatives.

Generally, the compound is at concentrations lying in the range 0.1% to 10% in most cosmetic compositions. For example, WO 96/14840 describes analgesic compositions comprising up to 9% menthyl lacate, and US 5 602 178 relates to bath products that have no menthol, containing about 6% menthyl lactate. JP 06329528 and JP 10231238 relate to compositions comprising menthol and menthyl lactate in ratios of 1/1 and 2/1 respectively, however at those ratios, those compositions do not deal with the problems associated with the secondary effects of menthol.

Some of the compositions described in the prior art documents can also contain fragrance reproducing the odor of mint. At low levels, mint odor also contributes to the sensation of cooling provided by menthyl lactate. Nevertheless, some of those fragrances can be expensive, and can give rise to other problems of skin tolerance.

Patent document US-A-5 523 078 discloses a cosmetic composition comprising 0.10 - 1.00 wt.% menthol and 0.50 - 3.00 wt.% menthyl lactate. This results in a menthol / menthyl lactate ratio of 1:30 - 2:1.

The object of the present invention is thus to provide a freshening composition, in particular a composition for cleaning the body, which is freshening and not irritating, and which also feels pleasant and provides a slight odor of freshness. One of the difficulties thus lies in developing a freshening composition based on menthol and on its derivatives while keeping the mint odor at a level that is sufficiently low. This makes it possible, optionally, to add other freshening fragrances that are less powerful and non-irritating.

Thus, no document of the prior art describes or suggests the present invention as defined here-below.

### Description

The present invention provides a freshening cosmetic composition characterized in that it comprises 0.01% to 2% by weight menthol and 0.1% to 10% by weight menthyl lactate, the menthol / menthyl lactate ratio lying in the range 1/1 to 1/10, and being such that the odor of the menthol is barely perceptible, said composition not being irritating, in particular for the sensitive parts of the human body. This object is more particularly achieved with a menthol / menthyl lactate ratio lying in the range 1/6 to 1/2. Preferably, the ratio is set at about 1/2.5, and the composition of the invention comprises 0.01% to 0.5% by weight of menthol, and 0.1% to 1% by weight of menthyl lactate.
Advantageously, the composition comprises 0.08% to 0.12% by weight of menthol and 0.2% to 0.3% of menthyl lactate.

No irritation spoils the beneficial freshening effect obtained by associating menthol and menthyl lactate in the concentrations and ratios specified above.

In addition, the "mint" odor due to the menthol is only faintly perceptible, so the composition can further include at least one fragrance, that can be different from or similar to the fragrance of "mint". In this respect, particular mention can be made of the fragrance "glacier SG 809 A" which reproduces the feeling and the odor of freshness at a concentration of 0.5% to 5% by weight, but without being overpowering and/or irritating.

The general formula of menthyl lactate is as follows:

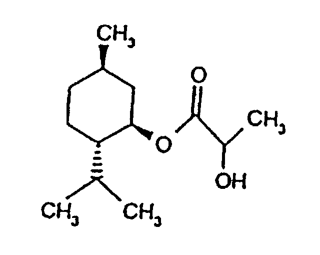

This compound contains four asymmetrical carbon atoms. It therefore exists in 16 different stereoisomers. In the context of the invention, the term "menthyl lactate" thus covers not only all such stereoisomers, but also any racemate or mixture of said stereoisomers.

The composition of the invention can also comprise at least one surface-active agent selected from anionic surface-active agents, preferably sodium cocoyl isethionate, at a concentration in the range 1% to 10% by weight, at least one thickening agent selected in particular from xantham gums, carbomers, preferably hydroxypropyl methylcellulose at a concentration lying in the range 0.2% to 10% by weight, and at least one conditioning polymer at a concentration lying in the range 0.25% to 2% by weight.

The said composition can also comprise a lustering agent, preferably a mixture of sodium laureth sulfate and of glycol distearate, at a concentration lying in the range 0.1% to 1% by weight, a foaming agent, preferably lauryl betaine, at a concentration lying in the range 1% to 10% by weight, an association of surface active agents such as sodium laureth sulfate, ammonium laureth sulfate, lauryl glucoside, such that the total concentration of surface active agent in the composition lies in the range 5% to 30%, and preferably in the range 15% to 25% by weight, and/or at least one hydrating agent, in particular glycerol and/or polyethylene glycol, at a concentration lying in the range 1% to 20% by weight.

Naturally, the composition of the invention is not limited to the ingredients mentioned above. For example, it can also contain a buffer, a dye, an anti-oxidant, and/or a preservative.

In a preferred implementation of the present invention, the composition is intended for use as a shower gel.
In this case, the composition of the invention may have the following compounds in percentage by weight:
- Water 63,15 %
- Sodium laureth sulfate 12 %
- Hydroxypropyl methylcellulose 0,5 %
- Sodium cocoyl isethionate 7 %
- Ammonium lauryl sulfate 3 %
- Lauryl glucoside 1 %
- Lauryl betaine 4 %
- Polyquaternium-10 5 %
- Polyethylene glycol (PEG) 2 %
- Preservative(s) 0,5 %
- Fragrance 1,5 %
- Menthyl lactate 0,25 %
- Menthol 0,1 %
   The present invention also provides the use of a cooling composition comprising from 0.1 to 2 wt % menthol and from 0.1 to 10 wt % menthyl lactate, the menthol/menthyl lactate ratio lying in the range 1/1 to 1/10 and being such that the odor of menthol is barely perceptible, said composition not being irritating, in particular for the sensitive parts of the human body, as a bathing product, in particular as a two-in-one product for simultaneously cleaning and freshening the skin.
   The present invention also provides the use of the above-defined composition as a product for washing with, in particular as a two-in-one product for simultaneously cleaning and freshening the skin, in particular as a shower gel or as a foaming gel for the face.

### Example 1: freshening shower gel with pH 5.5

The preferred shower gel of the invention comprises the following compounds in percentage by weight:

| **Compounds** | **% Activity** | **% by weight** | **% by weight active** |
|---|---|---|---|
| - Water | | 63,15 | |
| - Sodium laureth sulfate | 70 | 12 | 8,4 |
| - Hydroxypropyl methylcellulose | 100 | 0,5 | 0,5 |
| - Sodium cocoyl isethionate | 100 | 7 | 7 |
| - Ammonium lauryl sulfate | 70 | 3 | 2,1 |
| - Lauryl glucoside | 50 | 1 | 0,5 |
| - Lauryl betaine | 30 | 4 | 1,2 |
| - Polyquaternium-10 | 10 | 5 | 0,5 |
| - Polyethylene glycol (PEG) | 100 | 2 | 2 |
| - Preservative(s) | 100 | 0,5 | 0,5 |
| - Fagrance | 100 | 1,5 | 1,5 |
| - Menthyl lactate | 100 | 0,25 | 0,25 |
| - Menthol | 100 | 0,1 | 0,1 |

### Example 2: comparison of the feeling of freshness obtained with various products (refreshing shower gel at pH 5.5)

**Table 1:**

| Products tested | | | | |
|---|---|---|---|---|
| Product | Isopulegol | Menthol | Menthyl lactate | "Glacier" fragrance |
| A | 0.5% | 0.08% | | 0.5% |
| B | | 0.08% | 0.5% | 1.0% |
| C | | 0.1% | 0.25% | 1.5% |
| D | | 0.08% | 0.25% | 2% |

Experimental protocol (blind test):
Products A, B, C, and D were applied to the feet of individuals in a confidential study. 1 ml of each product was spread over the total surface area of a foot, and contact with the product was set at 2 minutes. The product was then rinsed off using warm water; and the feet were dried. The control used in this study consisted in immersing the other foot in water at ambient temperature, and also in water at 4°C for checking the "very cold" parameter. The test was repeated every day for several days.

The refreshing effect of said compositions was evaluated on a linear scale going from 0 (no effect) to 120 (very cold, like ice). This assessment was made immediately after the product had been applied (T0), after the feet had been rinsed and dried (T0'), and then every minute for 5 minutes. In addition to this assessment, an objective evaluation was performed by measuring the temperature of the skin of the feet.

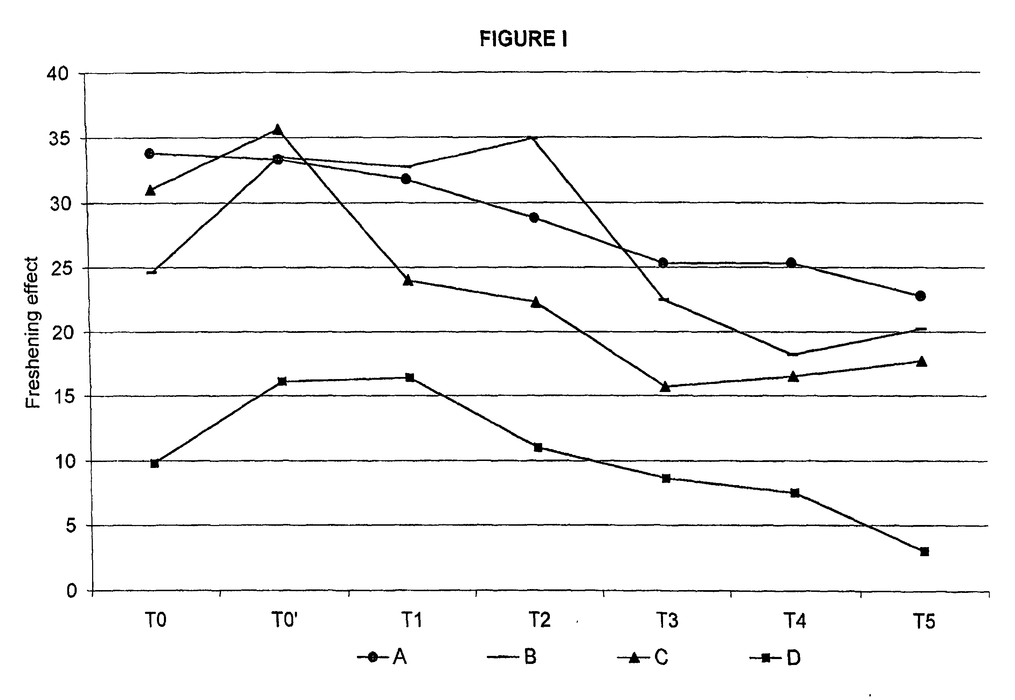

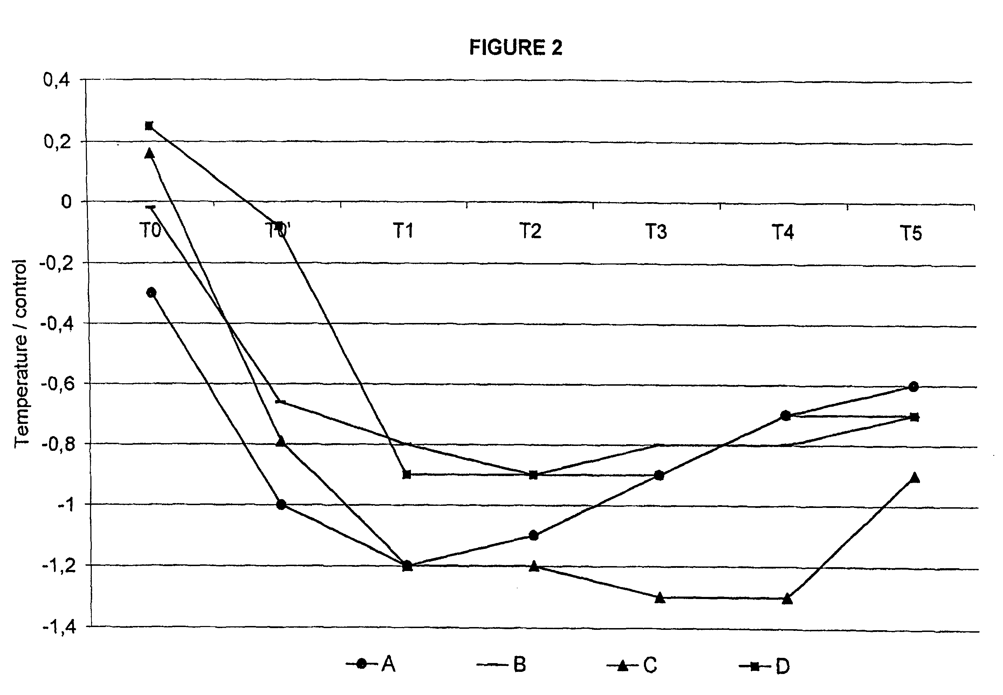

The results are given in Tables 2 and 3 below and in Figures 1 and 2 hereafter.

**Table 2:**

| Cooling effect compared with control | | | | | | | |
|---|---|---|---|---|---|---|---|
| | T0 | T0' | T1 | T2 | T3 | T4 | T5 |
| A | 33.8 | 33.3 | 31.8 | 28.8 | 25.3 | 25.3 | 22.8 |
| B | 24.6 | 33.5 | 32.7 | 34.9 | 22.5 | 18.2 | 20.2 |
| C | 31 | 35.6 | 24 | 22.3 | 15.7 | 16.5 | 17.7 |
| D | 9.8 | 16.1 | 16.4 | 11 | 8.6 | 7.5 | 3 |

**Table 3:**

| Temperature variation relative to control | | | | | | | |
|---|---|---|---|---|---|---|---|
| | T0 | T0' | T1 | T2 | T3 | T4 | T5 |
| A | -0.3 | -1 | -1.2 | -1.1 | -0.9 | -0.7 | -0.6 |
| B | -0.02 | -0.66 | -0.8 | -0.9 | -0.8 | -0.8 | -0.7 |
| C | 0.16 | -0.79 | -1.2 | -1.2 | -1.3 | -1.3 | -0.9 |
| D | 0.25 | -0.08 | -0.9 | -0.9 | -0.9 | -0.7 | -0.7 |

The result of this study shows that the optimum composition for obtaining the cooling effect corresponds to a menthol / menthyl lactate ratio set at about 1/2.5, with a menthol content by weight of about 0.08% to 0.12% and a menthyl lactate content by weight of 0.25%.

### Example 3: study on how the odor of the composition was assessed

This study set out to determine the optimum ratio between the "mint" scent and the "Glacier" scent in above-described compositions A, B, C, and D. It appears clearly from the assessment of the panel that the "mint" odor, which is very powerful, can give rise to negative feelings above a certain level. This difficulty is eliminated by the menthol / menthyl lactate ratios of the invention, and also by adding the "Glacier" fragrance.

### Example 4: in vitro study of eye irritation

Various formulations of the refreshing shower gel type composition having pH 5.5 have been tested to determine potential for irritating the eyes at various concentrations and ratios of menthol and menthyl lactate.
The results are given in Table 4 below.

**Table 4**

| Ingredient | Hetcam 5% | Hetcam 1% |
|---|---|---|
| Menthol 0.08% | 9.0±2.0 | 6.0±2.3 |
| | Irritant | Moderately irritant |
| Menthol 0.08% | 8.0±0.0 | 4.0±0.0 |
| Menthyl lactate 0.5% | Moderately irritant | Slightly irritant |
| Menthol 0.1% | 8.0±0.0 | 4.0±2.5 |
| Menthyl lactate 0.5% | Moderately irritant | Slightly irritant |

The combinations menthol 0.08% + menthyl lactate 0.5% and menthol 0.1% + menthyl lactate 0.5% which are effective for refreshing the skin are well tolerated by the most sensitive parts of the body.

### Example 5: study concerning skin tolerance

This study was performed to show that the skin tolerates repeated application of the shower gels of the invention.
Shower gels as described above, and also baby shampoo used as a reference, were applied to the antero-internal faces of the forearms.
The skin state of the application zone was verified and then humidified lightly using cotton wool moistened with warm water prior to applying the gels by massage that lasted for 10 seconds. A first reading was taken at T = 2 minutes. After rinsing in warm water and drying (using absorbant tissue paper), a second reading was performed at T = 10 minutes.
Every day the same application was repeated, and a new reading taken.

The results show that no undesirable effect (no burning sensation, no tingling, no itching, no erythema) occurred after using shower gels of the invention.

## Claims

1. A freshening cosmetic composition **characterized in that** it comprises 0.01% to 2% by weight menthol and 0.1% to 10% by weight menthyl lactate, the menthol / menthyl lactate ratio lying in the range 1/6 to 1/2, and being such that the odor of the menthol is barely perceptible, said composition not being irritating, in particular for the sensitive parts of the human body.

2. A composition according to claim 1, **characterized in that** the menthol / menthyl lactate ratio is about 1/2.5.

3. A composition according to claim 1 or 2, **characterized in that** it comprises 0.01% to 0.5% by weight of menthol and 0.1% to 1% by weight of menthyl lactate, preferably 0.08% to 0.12% by weight of menthol and 0.2% to 0.3% by weight of menthyl lactate.

4. A composition according to any one of claims 1 to 3, **characterized in that** it further includes at least one fragrance, in particular the "glacier SG 809 A" fragrance, at a concentration lying in the range 0.5% to 5% by weight.

5. A composition according to any one of claims 1 to 4, **characterized in that** it further comprises at least one surface active agent selected from anionic surface active agents, preferably sodium cocoyl isethionate, at a concentration lying in the range 1% to 10% by weight.

6. A composition according to claim 5, **characterized in that** it further comprises at least one thickening agent selected in particular from xanthan gums, carbomers, preferably hydroxypropyl methylcellulose, at a concentration lying in the range 0.2% to 10% by weight.

7. A composition according to claim 5 or 6, **characterized in that** it further comprises at least one conditioning polymer.

8. A composition according to any one of claims 5 to 7, **characterized in that** it further comprises a lustering agent, preferably a mixture of sodium laureth sulfate and of glycol distearate, at a concentration lying in the range 0.1% to 1% by weight.

9. A composition according to any one of claims 5 to 8, **characterized in that** it further comprises a foaming agent, preferably lauryl betaine, at a concentration lying in the range 1% to 10% by weight.

10. A composition according to any one of claims 5 to 9, **characterized in that** it further comprises an association of surface active agents such as sodium laureth sulfate, ammonium laureth sulfate, lauryl glucoside, such that the total concentration in surface active agent in the composition lies in the range 5% to 30%, and preferably in the range 15% to 25%, by weight.

11. A composition according to any one of claims 5 to 10, **characterized in that** it further comprises at least one hydrating agent, in particular glycerol and/or polyethylene glycol, at a concentration lying in the range 1% to 20% by weight.

12. A composition according to any one of claims 5 to 11, **characterized in that** it further comprises at least one buffer, and/or a preservative.

13. A composition according to any one of claims 5 to 12, for use as a shower gel.

14. A composition according to any one of claims 5 to 13, **characterized in that** it comprises the following compounds in percentage by weight:
- Water 63,15 %
- Sodium laureth sulfate 12 %
- Hydroxypropyl methylcellulose 0,5 %
- *Sodium cocoyl isethionate* 7 %
- Ammonium lauryl sulfate 3 %
- Lauryl glucoside 1 %
- Lauryl betaine 4 %
- Polyquaternium-10 5 %
- Polyethylene glycol (PEG) 2 %
- Preservative(s) 0,5 %
- Fragrance 1,5 %
- Menthyl lactate 0,25 %
- Menthol 0,1 %

15. Use of a cooling composition comprising from 0.1 to 2 wt % menthol and from 0.1 to 10 wt % menthyl lactate, the menthol/menthyl lactate ratio lying in the range 1/1 to 1/10 and being such that the odor of menthol is barely perceptible, said composition not being irritating, in particular for the sensitive parts of the human body, as a bathing product, in particular as a two-in-one product for simultaneously cleaning and freshening the skin.

16. The use of the composition according to any one of claims 1 to 14, as a bathing product, in particular as a two-in-one product for simultaneously cleansing and freshening the skin.

17. A use according to one of claims 15 and 16 as a shower gel.

18. A use according to one of claims 15 and 16 as a foaming gel for the face.

## Revendications

1. Composition cosmétique rafraîchissante **caractérisée en ce qu'**elle comprend de 0,01 % à 2 % en poids de menthol et de 0,1 à 10 % en poids de lactate de menthyle, le ratio entre le menthol et le lactate de menthyle étant compris dans la gamme allant de 1 pour 6 à 1 pour 2 et étant tel que l'odeur du menthol est a peine perceptible, ladite composition n'étant pas irritante, en particulier pour les parties sensibles du corps humain.

2. Composition selon la revendication 1, **caractérisée en ce que** le ratio entre le menthol et le lactate de menthyle est d'environ 1 pour 2,5.

3. Composition selon la revendication 1 ou 2, **caractérisée en ce qu'**elle comprend de 0,01 % à 0,5 % en poids de menthol et de 0,1 % à 1 % en poids de lactate de menthyle, de préférence de 0,08 % à 0,12 % en poids de menthol et de 0,2 % à 0,3 % en poids de lactate de menthyle.

4. Composition selon l'une quelconque des revendications 1 à 3, **caractérisée en ce qu'**elle inclut également au moins un parfum, en particulier le parfum "glacier SG 809 A", à une concentration comprise dans la gamme allant de 0,5 % à 5 % en poids.

5. Composition selon l'une quelconque des revendications 1 à 4, **caractérisée en ce qu'**elle comprend en outre au moins un agent tensioactif sélectionné à partir des agents tensioactifs anioniques, de préférence du cocoyle iséthionate de sodium, à une concentration comprise dans la gamme allant de 1 % à 10 % en poids.

6. Composition selon la revendication 6, **caractérisée en ce qu'**elle comprend en outre au moins un agent épaississant sélectionné en particulier à partir des gommes de xanthane, des carbomères, de préférence l'hydroxypropyle cellulose, à une concentration comprise dans la gamme allant de 0,2 % à 10 % en poids.

7. Composition selon la revendication 5 ou 6, **caractérisée en ce qu'**elle comprend en outre au moins un polymère de conditionnement.

8. Composition selon l'une quelconque des revendications 5 à 7, **caractérisée en ce qu'**elle comprend en outre un agent lustrant, de préférence un mélange de laureth sulfate de sodium et de distéarate de glycérol, à une concentration comprise dans la gamme allant de 0,1 % à 1 % en poids.

9. Composition selon l'une quelconque des revendications 5 à 8, **caractérisée en ce qu'**elle comprend en outre un agent moussant, de préférence de la bétaine de lauryle, à une concentration comprise dans la gamme allant de 1 % à 10 % en poids.

10. Composition selon l'une quelconque des revendications 5 à 9, **caractérisée en ce qu'**elle comprend en outre une association d'agents tensioactifs tels que du laureth sulfate de sodium, du laureth sulfate d'ammonium, du glucoside de lauryle, de telle sorte que la concentration totale en agent tensioactif dans la composition soit comprise dans la gamme allant de 5 % à 30 %, de préférence dans la gamme allant de 15 % à 25 % en poids.

11. Composition selon l'une quelconque des revendications 5 à 10, **caractérisée en ce qu'**elle comprend en outre au moins un agent hydratant, en particulier du glycérol et/ou du polyéthylène glycol, à une concentration comprise dans la gamme allant de 1 % à 20 % en poids.

12. Composition selon l'une quelconque des revendications 5 à 11, **caractérisée en ce qu'**elle comprend en outre au moins un tampon et/ou un conservateur.

13. Composition selon l'une quelconque des revendications 5 à 12, destinée à être utilisée en tant que gel pour la douche.

14. Composition selon l'une quelconque des revendications 5 à 13, **caractérisée en ce qu'**elle comprend les composés suivants, en pourcentages en poids :
| | |
|---|---|
| Eau | 63,15% |
| Laureth sulfate de sodium | 12 % |
| Hydroxypropyle méthylcellulose | 0,5 % |
| Cocoyle iséthionate de sodium | 7 % |
| Lauryle sulfate d'ammonium | 3 % |
| Glucoside de lauryle | 1 % |
| Bétaine de lauryle | 4 % |
| Polyquaternium - 10 | 5 % |
| Polyéthylène glycol (PEG) | 2 % |
| Conservateur(s) | 0,5 % |
| Parfum | 1,5 % |
| Lactate de menthyle | 0,25 % |
| Menthol | 0,1 % |

15. Utilisation d'une composition rafraîchissante comprenant de 0,1 % à 2 % en poids de menthol et de 0,1 à 10 % en poids de lactate de menthyle, le ratio entre le menthol et le lactate de menthyle étant compris dans la gamme allant de 1 pour 1 à 1 pour 10 et étant tel que l'odeur du menthol est à peine perceptible, ladite composition n'étant pas irritante en particulier pour les parties sensibles du corps humain, en tant qu'un produit pour le bain, en particulier un produit deux en un pour nettoyer et rafraîchir la peau simultanément.

16. Utilisation d'une composition selon l'une quelconque des revendications 1 à 14, en tant qu'un produit pour le bain, en particulier un produit deux en un pour nettoyer et rafraîchir la peau simultanément.

17. Utilisation selon l'une des revendications 15 et 16, en tant qu'un gel pour la douche.

18. Utilisation selon l'une des revendications 15 et 16, en tant qu'un gel moussant pour le visage.

## Patentansprüche

1. Belebende kosmetische Zusammensetzung, **dadurch gekennzeichnet, dass** sie 0,01 bis 2 Gewichts-% Menthol und 0,1 bis 10 Gewichts-% Menthyllactat umfasst, wobei das Menthol/Menthyllactat-Verhältnis im Bereich von 1/6 bis 1/2 liegt und derart ist, dass der Geruch des Menthols kaum wahrnehmbar ist, wobei die Zusammensetzung insbesondere für die empfindlichen Teile des menschlichen Körpers nicht reizend ist.

2. Zusammensetzung nach Anspruch 1, **dadurch gekennzeichnet, dass** das Menthol/Menthyllactat-Verhältnis etwa 1/2,5 beträgt.

3. Zusammensetzung nach Anspruch 1 oder 2, **dadurch gekennzeichnet, dass** sie 0,01 bis 0,5 Gewichts-% Menthol und 0,1 bis 1,0 Gewichts-% Menthyllactat, vorzugsweise 0,08 bis 0,12 Gewichts-% Menthol und 0,2 bis 0,3 Gewichts-% Menthyllactat umfasst.

4. Zusammensetzung nach irgendeinem der Ansprüche 1 bis 3, **dadurch gekennzeichnet, dass** sie weiter mindestens einen Duftstoff, insbesondere den Duftstoff "glacier SG 809 A", bei einer Konzentration, die im Bereich von 0,5 bis 5 Gewichts-% liegt, einschließt.

5. Zusammensetzung nach irgendeinem der Ansprüche 1 bis 4, **dadurch gekennzeichnet, dass** sie weiter mindestens ein oberflächenaktives Mittel, das aus anionischen oberflächenaktiven Mitteln, vorzugsweise Natriumcocoylisethionat, ausgewählt ist, bei einer Konzentration, die im Bereich von 1 bis 10 Gewichts-% liegt, umfasst.

6. Zusammensetzung nach Anspruch 5, **dadurch gekennzeichnet, dass** sie weiter mindestens ein Verdickungsmittel, das insbesondere aus Xanthangummis, Carbomeren, vorzugsweise Hydroxypropylmethylcellulose, ausgewählt ist, bei einer Konzentration, die im Bereich von 0,2 bis 10 Gewichts-% liegt, umfasst.

7. Zusammensetzung nach Anspruch 5 oder 6, **dadurch gekennzeichnet, dass** sie weiter mindestens ein feuchtigkeitsregulierendes Polymer umfasst.

8. Zusammensetzung nach irgendeinem der Ansprüche 5 bis 7, **dadurch gekennzeichnet, dass** sie mindestens ein einen Schimmer verleihendes Mittel, bevorzugt eine Mischung aus Natriumlaurethsulfat und Glycoldistearat, bei einer Konzentration, die im Bereich von 0,1 bis 1 Gewichts-% liegt, umfasst.

9. Zusammensetzung nach irgendeinem der Ansprüche 5 bis 8, **dadurch gekennzeichnet, dass** sie weiter ein schäumendes Mittel, vorzugsweise Laurylbetain, bei einer Konzentration, die im Bereich von 1 bis 10 Gewichts-% liegt, umfasst.

10. Zusammensetzung nach irgendeinem der Ansprüche 5 bis 9, **dadurch gekennzeichnet, dass** sie weiter eine Verbindung von oberflächenaktiven Mitteln, wie Natriumlaurethsulfat, Ammoniumlaurethsulfat, Laurylglucosid, umfasst, derart, dass die Gesamtkonzentration an oberflächenaktiven Mitteln in der Zusammensetzung im Bereich von 5 bis 30 und vorzugsweise im Bereich von 15 bis 25 Gewichts-% liegt.

11. Zusammensetzung nach irgendeinem der Ansprüche 5 bis 10, **dadurch gekennzeichnet, dass** sie weiter mindestens ein Hydratisierungsmittel, insbesondere Glycerin und/oder Polyethylenglycol, bei einer Konzentration, die im Bereich von 1 bis 20 Gewichts-% liegt, umfasst.

12. Zusammensetzung nach irgendeinem der Ansprüche 5 bis 11, **dadurch gekennzeichnet, dass** sie weiter mindestens einen Puffer und/oder ein Konservierungsmittel umfasst.

13. Zusammensetzung nach irgendeinem der Ansprüche 5 bis 12 zur Verwendung als Duschgel.

14. Zusammensetzung nach irgendeinem der Ansprüche 5 bis 13, **dadurch gekennzeichnet, dass** sie die folgenden Verbindungen in Gewichtsprozenten umfasst:
- Wasser 63,15%
- Natriumlaurethsulfat 12%
- Hydroxypropylmethylcellulose 0,5%
- Natriumcocoylisethionat 7%
- Ammoniumlaurylsulfat 3%
- Laurylglucosid 1%
- Laurylbetain 4%
- Polyquatemium-10 5%
- Polyethylenglycol (PEG) 2%
- Konservierungsmittel 0,5%
- Duftstoff 1,5%
- Menthyllactat 0,25%
- Menthol 0,1%

15. Verwendung einer kühlenden Zusammensetzung, umfassend 0,1 bis 2 Gewichts-% Menthol und 0,1 bis 10 Gewichts-% Menthyllactat, wobei das Menthol/Menthyllactat-Verhältnis im Bereich von 1/1 bis 1/10 liegt und derart ist, dass der Geruch von Menthol kaum wahrnehmbar ist, wobei die Zusammensetzung insbesondere für die empfindlichen Teile des menschlichen Körpers nicht reizend ist, als Badeprodukt, insbesondere als Zwei-in-Eins-Produkt zur gleichzeitigen Reinigung und Belebung der Haut.

16. Verwendung der Zusammensetzung nach irgendeinem der Ansprüche 1 bis 14 als Badeprodukt, insbesondere als Zwei-in-Eins-Produkt zur gleichzeitigen Reinigung und Belebung der Haut.

17. Verwendung nach einem der Ansprüche 15 und 16 als Duschgel.

18. Verwendung nach einem der Ansprüche 15 und 16 als Schaumgel für das Gesicht.
